# EUROPEAN PATENT APPLICATION

(11) **EP 2 679 496 A2**
(43) Date of publication of application: **01.01.2014**
(21) Application number: 13174007.8
(22) Date of filing: 27.06.2013
(51) Int. Cl.: B64D 11/00, B64D 47/08, A62B 7/14

(54) **Passenger service unit with gesture control**

(30) Priority: 28.06.2012 US 201261665486 P
(71) Applicant: INTERTECHNIQUE, 78373 Plaisir Cedex (FR)
(72) Inventor: Hoffmann, Frederik, 23617 Stockelsdorf (DE); Boomgarden, Günter, 23684 Scharbeutz (DE); Rittner, Wolfgang, 23623 Ahrensbök (DE); Meckes, Rüdiger, 23919 Berkenthin (DE); Hollm, Marco, 25548 Rosdorf (DE)
(74) Representative: Eisenführ Speiser

(57) **Abstract**

The invention relates to a passenger service unit 10 for installation inside an aircraft cabin comprising a passenger service device, a control unit, coupled to the passenger service device for transmitting control signals comprising an imaging device 20 oriented to acquire a sequence of images out of a predetermined observed area and an image analysis device 40 coupled for signal transmission to said imaging device and adapted to analyze a static geometric structure within said observed area out of a single image of said imaging device.

## Description

The invention relates to a passenger service unit for installation inside an aircraft cabin comprising a passenger service device, a control unit, coupled to the passenger service device for transmitting control signals.

Such passenger service units are usually installed in the upper cabin part of an aircraft above the passenger seats on the backside of a passenger seat. Such passenger service units are required to provide service to the passengers and therefore include a single or a plurality of passenger service devices. Such passenger service device may be a reading light, a ventilation device, a cabin attendant call sign, a loud speaker, a display or e.g. an emergency oxygen device.

It is generally known to provide a user interface like a switch or a wheel or the like to act as a control unit for activating and controlling such passenger service devices. It is further known to provide a central control unit for activating particular passenger service devices like an emergency oxygen device to safely activate such emergency oxygen device in case of an emergency situation.

Whereas such user interfaces may provide a safe and reliable interaction of the user with the passenger service devices of the passenger service unit it is sometimes observed that passengers are not able to control passenger service devices in situations where the passengers are in a condition of danger, emergency or panic. In such situations, the passenger may not receive relevant services out of the passenger service unit required for him to provide important service and assistance for vital functions.

It is an object of the present invention to provide an improved passenger service unit which is able to safely provide service and assistance in such situations of danger, emergency and panic.

This problem is solved by providing a passenger service unit of the type explained beforehand, further comprising
- an imaging device oriented to acquire a sequence of images out of a predetermined observed area and
- an image analysis device coupled for signal transmission to said imaging device and adapted to analyze a static geometric structure within said observed area out of a single image of said imaging device or a dynamic movement out of sequence of images of said imaging device,
- wherein said image analysis device comprises a storage device wherein at least one predetermined static geometric structure or predetermined dynamic movement and a control signal associated with said predetermined static geometric structure or predetermined dynamic movement, respectively, is stored and
- wherein said image analysis device comprises a comparator unit for comparing said static geometric structure or said dynamic movement acquired by said imaging device with said predetermined static geometric structure or predetermined dynamic movement,
- wherein said image analysis device is coupled to said control unit for signal transmission and is adapted to send said control signal associated with said predetermined static geometric structure or predetermined dynamic movement if said static geometric structure or said dynamic movement acquired by said imaging device coincides with said predetermined static geometric structure or predetermined dynamic movement, respectively, to said service device.

According to the invention, an imaging device and an image analysis device is provided to acquire and analyze an image or a sequence of images out of the region where the passenger is sitting. This imaging device can be a digital camera. The imaging device is preferably positioned within the cabin of the aircraft in an upper region, e.g. mounted in a ceiling compartment as part of a passenger service unit arranged in such a ceiling compartment. The imaging device is oriented towards the passenger seat and has an angle of view which is sufficient to acquire an image of the face and the hands of the passenger preferably.

The imaging device may be adapted to permanently observe the passenger to acquire any static geometric structure or dynamic movement in a sequence of images of the passenger. Such sequence of images is transmitted to the image analysis device which may be a computer or electronic processing unit being programmed with a software for analyzing images. The image analysis device is able to identify static geometric structures or dynamic movements of such geometric structures. For example, the image analysis device may be able to identify the eye region or the mouth region of a face of a passenger and any movements and geometric changes of the eye region or the mouth region. Further, the image analysis device may be adapted to identify a hand or both hands of a passenger and one or both arms of a passenger and movements of the hands, the fingers or the arms. By identifying such geometric structures and movements of such geometric structures a specific scheme of movements is recognized by the image analysis device. Such movement or geometric structure is hereafter compared with predetermined geometric structures or dynamic movements which are stored in a storage device. Such storage device may be an electronic storage device like a RAM, ROM or hard disk or the like. In the storage device a single, preferably a plurality of static geometric structures or dynamic movement are stored and associated with a particular control signal or a plurality of particular control signals, respectively. In case that a static geometric structure or a dynamic movement is found to be identical or similar to such predetermined static geometric structure or dynamic movements the control signal associated with such static geometric structure or dynamic movement is identified, read out of the storage device and sent to the control unit for effecting an action at a passenger service device.

The imaging device may be sensor arrangement for detecting a three-dimensional geometry of an object. In particular the imaging device may be a time of flight camera. Such time-of-flight camera may be adapted to detect intensity of light of a plurality of surface sections of an object defining a gesture out of an imaging area and further adapted to detect runtime of a light pulse from the camera to and from said plurality of surface sections of said object inside said area to thus calculate respective distances between the camera and said surface sections. The time-of-flight camera is then adapted to calculate a threedimensional geometry of said object surface out of said intensity of light and said distance.

By this, particular actions taken by the passenger e.g. movements or facial expressions expressing a panic situation or any other needs of the passenger can be interpreted and an adequate action and provision of services out of the passenger service device can be started in reaction thereto. By this, an immediate action of the passenger service device is possible without the need of the passenger to conduct a specific, controlled manual interaction with a user interface or the like. Thus, the passenger service unit according to the invention provides a much safer and reliable interaction between the passenger and the passenger service device and provides a safe reaction of the passenger service device to any needs of the passenger.

It is particularly preferred that said passenger service device comprises an emergency oxygen device for providing oxygen to a passenger in an emergency situation. An emergency oxygen device is required by the passenger in a critical emergency situation like a decompression situation on board of an aircraft. Such decompression situation is often accompanied by fog or smoke within the cabin producing a panic reaction of the passenger or the least adversely affecting rationale behavior of the passenger. It is particularly relevant to immediately provide oxygen to the passenger via an oxygen mask to the passenger in such a situation. The passenger service unit according to the invention may safely activate an emergency oxygen device providing the oxygen mask to the passenger and starting oxygen supply in reaction to specific behavior of the passenger acquired by the imaging device and recognized by the image analysis device.

It is further preferred that said predetermined static geometric structure or predetermined dynamic movement is a gesture, said gesture being selected from a facial expression, a hand sign or a sequence of hand signs. It has been shown that in particular a facial expression, a hand sign or a sequence of hand signs may be used as a gesture for being acquired by the imaging device and analyzed by the image analysis device to derive therefrom a specific need of the passenger to put out a control signal to the control unit. It is to be understood that a combination of any of the three gestures may be used as well for producing a unique gesture relating to such a control signal.

It is further preferred that said image analysis device is adapted to identify a gesture out of an image acquired by said imaging device and wherein at least one predetermined gesture and a control signal associated with said gesture is stored in said storage device and wherein said image analysis device is adapted to send said control signal associated with said predetermined gesture if said gesture acquired by said imaging device is a identical or similar said predetermined gesture to said service device. By identifying such specific gestures a safe and quick control of passenger service devices is possible.

According to a further preferred embodiment said image analysis device is adapted to identify and differentiate a left hand and a right hand and to process gestures of the left hand, the right hand and/or gestures comprising a combination of hand signs or sequences of hand signs of the left and the right hand. According to this embodiment, gestures of the left hand can be differentiated from gestures of the right hand and further gestures comprising a combination of a static geometric structure or a dynamic movement of the left and the right hand can be identified and used to activate a specific control signal. It is to be understood that each gesture of the left hand and the right hand and each such combination may be associated to a unique control signal. Alternatively, a gesture of the left hand and a similar gesture of the right hand may be associated to a common control signal to allow the passenger to alternatively use the left or the right hand for effecting such a control signal.

According to a further preferred embodiment the passenger service unit according to the invention comprises a first passenger service device for providing service to a first passenger and a second passenger service device for providing service to a second passenger, wherein said image analysis device is adapted to identify a first face associated with said first passenger and a second face associated with said second passenger out of the image acquired by said imaging device wherein said image analysis device is adapted to associate a gesture to either the first face or the second face and adapted to send said control signal associated with said gesture to the first passenger service device if the gesture is associated with the first face and to send said control signal associated with said gesture to the second passenger service device if the gesture is associated with the second face. In this particular embodiment two passenger service devices are provided and arranged in such a way that each passenger service device provides a service to a passenger associated to said passenger service device. Thus, two passengers are serviced individually out of the passenger service unit.

The imaging device and the image analysis device is in this case adapted to acquire an image of both passengers and to identify and follow a face of the passengers to associate any gesture within the sequence of images to any of the two passengers. By this, it is possible to provide a service to any of the two passengers using a centralized imaging device and image analysis device out of one image or sequence of images acquired and containing both passengers.

According to a further preferred embodiment the passenger service unit further comprises a third passenger service device and optionally further passenger service devices, wherein said image analysis device is adapted to further identify a third face associated with a third passenger and optional a further face associated with a further passenger and is adapted to associate a gesture to said third first face or the further face and is adapted to send said control signal associated with said gesture to the third passenger service device or the further passenger service device if the gesture is associated with the third face or the further face, respectively. According to this embodiment at least three passenger service devices are comprised in the passenger service unit and provide services to at least passengers, respectively. The imaging device and the image analysis device is in this case adapted to identify three faces of the passengers and associate any gestures of said face or a hand associated to such face to selectively provide a control signal to a specific passenger service device servicing the passenger corresponding to said face.

According to a further preferred embodiment a housing for accommodating said passenger service device(s), said control unit and said imaging device is provided. This allows for an integral arrangement of those components required for the service, image acquisition and control of the passenger service devices within one unit to facilitate installation on board of an aircraft.

It is further preferred that said imaging device comprises two separate imaging units arranged at a distance from each other and an imaging processor for processing said images out of said two imaging units to generate 3D-information out of said images, wherein said 3D information is provided to said image analysis device. This embodiment allows for three-dimensional recognition of gestures and thus more precision with regard to recognition and comparison of such gestures to predetermined gestures stored in the storage device. It is preferred that said two imaging devices are comprised in the passenger service unit. However, in specific applications it may be preferred to provide a single image analysis in a passenger service unit cooperating with a second imaging device arranged in another passenger service unit which is mounted adjacent to said passenger service unit.

According to a further preferred embodiment said control signal is a signal selected from an activation signal for activating an emergency oxygen device, a switch on/off signal for switching on and off a reading light, a call signal for providing a cabin attendant call to a signalizing unit, an increase signal for increasing a ventilation of a ventilation device or a decrease signal for decreasing a ventilation of a ventilation device. According to this embodiment, the gestures of the passenger are used to provide control signals for a single or a number of relevant services in regular flight condition an emergency situation on board of an aircraft. It is to be understood that the passenger service unit according to the invention in particular may be adapted to provide two, three, four or five different control signals for effecting a plurality of said functions by associating different predetermined gestures to the control signals such that each control signal is associated with a unique gesture.

According to a further preferred embodiment the passenger service unit may be further improved in such a way that in said storage device a plurality of predetermined different static geometric structures or predetermined dynamic movements and a respective plurality of different control signals is stored, each control signal being associated with one predetermined static geometric structure or predetermined dynamic movement, respectively. This embodiment allows to conveniently and safely effect different services to the passenger by geometric structures or dynamic movements like e.g. gestures of a facial expression, a single or both hands of the passenger.

A further aspect of the invention is a method for controlling a passenger service device in a passenger service unit inside an aircraft cabin comprising the steps:
- acquiring a sequence of images out of a predetermined observed area using an imaging device transmitting said sequence of images to an image analysis device,
- analyzing a static geometric structure within said observed area out of a single image of said sequence of images or a dynamic movement out of said sequence of images using said imaging analysis device, wherein said image analysis device comprises a storage device wherein at least one predetermined static geometric structure or predetermined dynamic movement and a control signal associated with said predetermined static geometric structure or predetermined dynamic movement, respectively, is stored and

- comparing said static geometric structure or said dynamic movement acquired by said imaging device with said predetermined static geometric structure or predetermined dynamic movement using a comparator unit comprised in said image analysis device,
- sending said control signal associated with said predetermined static geometric structure or predetermined dynamic movement if said static geometric structure or said dynamic movement acquired by said imaging device coincides with said predetermined static geometric structure or predetermined dynamic movement, respectively, to said service device.

The method can be further improved in such a way that said passenger service device comprises an emergency oxygen device for providing oxygen to a passenger in an emergency situation and said emergency oxygen device is activated by said control sign.

The method may be further improved in such a way that said predetermined static geometric structure or predetermined dynamic movement is a gesture, said gesture being selected from a facial expression, a hand sign or a sequence of hand signs.

Still further, the method may be improved in such a way that said passenger service unit comprises a first passenger service device for providing service to a first passenger and a second passenger service device for providing service to a second passenger, further comprising the steps of identifying a first face associated with said first passenger and a second face associated with said second passenger out of the image acquired by said imaging device using said image analysis device, associating, by said image analysis device, a gesture to either the first face or the second face and sending said control signal associated with said gesture to the first passenger service device if the gesture is associated with the first face and sending said control signal associated with said gesture to the second passenger service device if the gesture is associated with the second face.

Finally, the method may be further improved in such a way that said control signal activates an emergency oxygen device, switches on and off a reading light, provides a cabin attendant call to a signalizing unit, increases a ventilation of a ventilation device or decreases a ventilation of a ventilation device.

With regard to the so disclosed method and preferred embodiment of the method reference is made to the above description of the passenger service unit being adapted to conduct the respective method, the specific embodiments thereof and the advantages described in connection thereto. A preferred embodiment is shown the figures.

A preferred embodiment of the invention is described with reference to the figures.
Figure 1 shows a schematic perspective view of a preferred embodiment according to the invention,
Figure 2 shows a schematic view of a detail of figure 1.

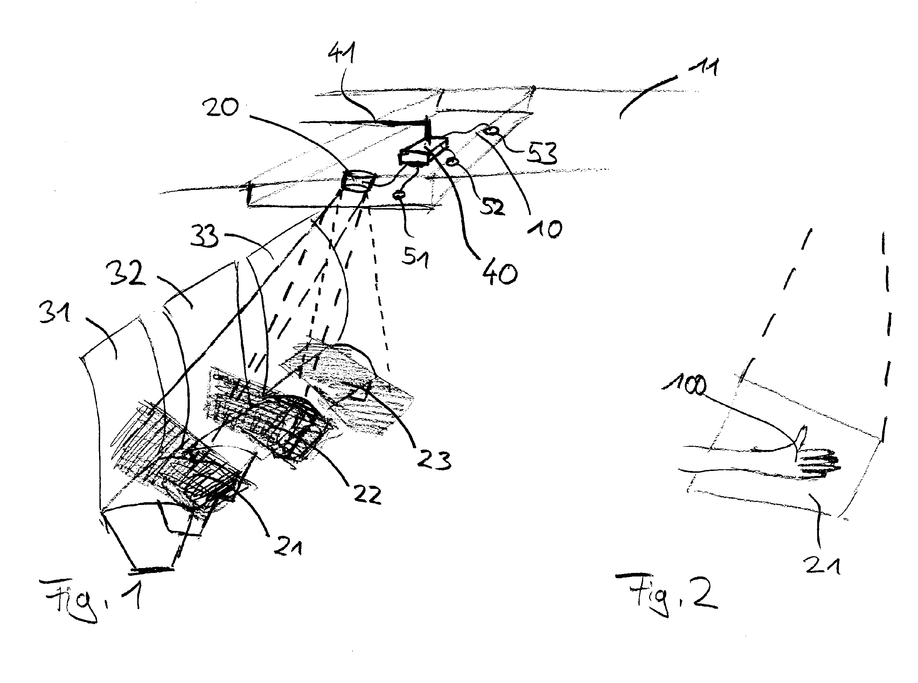

As can be seen in figure 1 a passenger service unit (PSU) 10 is moved to an overhead supply channel 11. The PSU may comprise an image acquiring device 20 which acquires image data from three distinct virtual zones 21, 22, 23.

These three virtual zones 21-23 are associated to three passenger seats 31, 32, 33 respectively and cover an area where a passenger sitting on these seats usually positions his hands.

The image acquiring device 20 may preferably be a time of flight camera capable to detect a three-dimensional surface in any of said virtual zones 21-23 and changes of such three dimensional surface.

The imaging acquiring device 20 is coupled to a processing device 40 for signal transmission to the processing device. The processing device 40 is adapted to process the image data provided by the image acquiring device 20 to recognize three-dimensional surfaces and to differentiate the position of such three-dimensional surfaces to be in either one of virtual zones 21, 22 or 23.

Further, the processing device 40 is adapted to identify changes of such three-dimensional surfaces like movements of objects incorporating such three-dimensional surfaces in a sequence of images acquired by the image acquiring device 20. Such movements may be relative movements of fingers of a hand 100 as shown in figure 2 or relative movements of two hands in relation to each other, rotation of such hands or any other gestures signalizing a specific geometry by the fingers or the hands or movements. Generally, it is to be understood that the invention is not limited to gestures, movements and signals of a single or two hands only and other gestures may be acquired and recognized by the image acquiring device 20 and the processing device 40 as well. In particular, movements of the passenger's head or body signalizing that the passenger feels inconvenient or signalizing unconsciousness of the passenger may be acquired and detected by the processing device as well.

The processing device 40 is coupled to a control line 41 which processes signals to and from the processing device 40 with a central processing unit which controls common functionalities of the aircraft like a cabin attendant call. Further, the processing device 40 is coupled to individual passenger service components comprised in the PSU 10 like reading lights 51-53 to activate such passenger service components depending on specific gestures determined by the processing unit. It is to be understood that reading light 51 associated to passenger seat 31 will be controlled depending on any gestures detected in virtual zone 21 only which is associated to the same passenger seat 31.

## Claims

1. A passenger service unit for installation inside an aircraft cabin comprising
- a passenger service device,
- a control unit, coupled to the passenger service device or the for transmitting control signals,
**characterized by**
- an imaging device oriented to acquire a sequence of images out of a predetermined observed area and
- an image analysis device coupled for signal transmission to said imaging device and adapted to analyze a static geometric structure within said observed area out of a single image of said imaging device or a dynamic movement out of sequence of images of said imaging device,
- wherein said image analysis device comprises a storage device wherein at least one predetermined static geometric structure or predetermined dynamic movement and a control signal associated with said predetermined static geometric structure or predetermined dynamic movement, respectively, is stored and
- wherein said image analysis device comprises a comparator unit for comparing said static geometric structure or said dynamic movement acquired by said imaging device with said predetermined static geometric structure or predetermined dynamic movement,
- wherein said image analysis device is coupled to said control unit for signal transmission and is adapted to send said control signal associated with said predetermined static geometric structure or predetermined dynamic movement if said static geometric structure or said dynamic movement acquired by said imaging device coincides with said predetermined static geometric structure or predetermined dynamic movement, respectively, to said service device

2. Passenger service unit according to claim 1,
Wherein said passenger service device comprises an emergency oxygen device for providing oxygen to a passenger in an emergency situation.

3. Passenger service unit according to claim 1 or 2,
Wherein said predetermined static geometric structure or predetermined dynamic movement is a gesture, said gesture being selected from
- a facial expression,
- a hand sign
- a sequence of hand signs.

4. Passenger service unit according to claim 3,
**characterized in that** said image analysis device is adapted to identify a gesture out of an image acquired by said imaging device and wherein at least one predetermined gesture and a control signal associated with said gesture is stored in said storage device and wherein said image analysis device is adapted to send said control signal associated with said predetermined gesture if said gesture acquired by said imaging device is identical or similar to said predetermined gesture to said service device.

5. Passenger service unit according to claim 3 or 4,
**characterized in that** said image analysis device is adapted to identify and differentiate a left hand and a right hand and to process gestures of the left hand, the right hand and/or gestures comprising a combination of hand signs or sequences of hand signs of the left and the right hand.

6. Passenger service unit according to any of the preceding claims 3-5, comprising
- a first passenger service device for providing service to a first passenger and
- a second passenger service device for providing service to a second passenger,
- wherein said image analysis device is adapted to identify a first face associated with said first passenger and a second face associated with said second passenger out of the image acquired by said imaging device
- wherein said image analysis device is adapted to associate a gesture to either the first face or the second face and adapted
○ to send said control signal associated with said gesture to the first passenger service device if the gesture is associated with the first face and
○ to send said control signal associated with said gesture to the second passenger service device if the gesture is associated with the second face.

7. Passenger service unit according to claim 6,
further comprising a third passenger service device and optionally further passenger service devices, wherein said image analysis device is adapted to further identify a third face associated with a third passenger and optional a further face associated with a further passenger and is adapted to associate a gesture to said third first face or the further face and is adapted ○ to send said control signal associated with said gesture to the third passenger service device or the further passenger service device if the gesture is associated with the third face or the further face, respectively.

8. Passenger service unit according to any of the preceding claims, Comprising a housing for accommodating said passenger service device, said control unit, and said imaging device.

9. Passenger service unit according to any of the preceding claims,
wherein said imaging device comprises two separate imaging units arranged at a distance from each other and an imaging processor for processing said images out of said two imaging units to generate 3D-information out of said images, wherein said 3D information is provided to said image analysis device.

10. Passenger service unit according to any of the preceding claims,
wherein said control signal is a signal selected from
- an activation signal for activating an emergency oxygen device,
- a switch on/off signal for switching on and off a reading light,
- a call signal for providing a cabin attendant call to a signalizing unit,
- an increase signal for increasing a ventilation of a ventilation device
- a decrease signal for decreasing a ventilation of a ventilation device.

11. Passenger service unit according to any of the preceding claims,
wherein in said storage device a plurality of predetermined different static geometric structures or predetermined dynamic movements and a respective plurality of different control signals is stored, each control signal being associated with one predetermined static geometric structure or predetermined dynamic movement, respectively.

12. A method for controlling a passenger service device in a passenger service unit inside an aircraft cabin comprising
**characterized by** the steps of
- acquiring a sequence of images out of a predetermined observed area using an imaging device
- transmitting said sequence of images to an image analysis device
- analyzing a static geometric structure within said observed area out of a single image of said sequence of images or a dynamic movement out of said sequence of images using said imaging analysis device,
- wherein said image analysis device comprises a storage device wherein at least one predetermined static geometric structure or predetermined dynamic movement and a control signal associated with said predetermined static geometric structure or predetermined dynamic movement, respectively, is stored and
- comparing said static geometric structure or said dynamic movement acquired by said imaging device with said predetermined static geometric structure or predetermined dynamic movement using a comparator unit comprised in said image analysis device,
- sending said control signal associated with said predetermined static geometric structure or predetermined dynamic movement if said static geometric structure or said dynamic movement acquired by said imaging device coincides with said predetermined static geometric structure or predetermined dynamic movement, respectively, to said service device.

13. Method according to claim 12,
Wherein said passenger service device comprises an emergency oxygen device for providing oxygen to a passenger in an emergency situation and said emergency oxygen device is activated by said control signal.

14. Method according to claim 12 or 13,
Wherein said predetermined static geometric structure or predetermined dynamic movement is a gesture, said gesture being selected from
- a facial expression,
- a hand sign
- a sequence of hand signs.

15. Method according to claim 14, wherein said passenger service unit comprises
- a first passenger service device for providing service to a first passenger and
- a second passenger service device for providing service to a second
passenger,
further comprising the steps of
- identifying a first face associated with said first passenger and a second face associated with said second passenger out of the image acquired by said imaging device using said image analysis device,
- associating, by said image analysis device, a gesture to either the first face or the second face and
○ sending said control signal associated with said gesture to the first passenger service device if the gesture is associated with the first face and
○ sending said control signal associated with said gesture to the second passenger service device if the gesture is associated with the second face.

16. Method according to any of the preceding claims 12-15,
wherein said control signal
- activates an emergency oxygen device,
- switches on and off a reading light,
- provides a cabin attendant call to a signalizing unit,
- increases a ventilation of a ventilation device
- decreases a ventilation of a ventilation device.
